# EUROPÄISCHE PATENTSCHRIFT

(11) **EP 1 529 542 B1**
(45) Veröffentlichungstag und Bekanntmachung des Hinweises auf die Patenterteilung: **01.02.2006**
(21) Anmeldenummer: 03025541.8
(22) Anmeldetag: 07.11.2003
(51) Int. Cl.: A61L 27/30, A61L 27/04

(54) **Gelenkendoprothese mit metallischem Kern und Glaskeramik-Beschichtung**
Joint endoprosthesis with metallic core and vitreous ceramic coating
Endoprothèse d'articulation avec un noyau métallique et un revêtement de céramique vitreuse

(43) Veröffentlichungstag der Anmeldung: 11.05.2005
(73) Patentinhaber: ESKA Implants GmbH & Co., 23556 Lübeck (DE)
(72) Erfinder: Grundei, Dr. Hans, 23558 Lübeck (DE)
(74) Vertreter: Fuchs Mehler Weiss & Fritzsche

(56) Entgegenhaltungen:
- CH-A- 681 423
- DE-A- 4 317 448
- GB-A- 1 451 283

## Beschreibung

Die Erfindung betrifft eine Gelenkendoprothese, welche ein Gelenkteil mit einem metallischen Kern aufweist, das zumindest bereichsweise an einem anderen Gelenkteil bei Ausübung der Gelenkfunktion abrollt und/oder abgleitet.

Typische Beispiele für eine solche Gelenkendoprothese sind ein künstliches Kniegelenk, ein Schultergelenk und vor allem ein künstliches Hüftgelenk.

In diesem Bereich sind dabei verschiedene Material-Paarungen seit langem bekannt, wie beispielsweise eine metallische Gelenkkugel mit einem Gleitpartner in Form eines Inlays aus Polyäthylen in einer Beckenpfanne, ebenso wie eine hartkeramische Gelenkkugel mit einem hartkeramischen oder einem komposit-keramischen Einsatz in einer Beckenpfanne.

Problematisch sind nach wie vor die Abriebserscheinungen bei einem der Gleitpartner. Diese sind zwar gering, jedoch messbar, was in der Regel nach einigen Jahren einen Austausch zumindest eines Gelenkteils erfordert. Dies ist nicht nur für den Patienten eine hohe Belastung, es stellt auch einen erheblichen Kostenfaktor dar.

Demgemäß ist es die Aufgabe der vorliegenden Erfindung, eine Gelenkendoprothese der eingangs genannten Art anzugeben, die langzeitstabil und verschleißfest ist und dabei eine hohe Festigkeit der Gelenkkomponenten besitzt. Darüber hinaus soll eine Paarung von Gelenkteilen angegeben werden, welche diese vorteilhaften Eigenschaften aufweist.

Gelöst wird diese Aufgabe durch eine Gelenkendoprothese mit den Merkmalen des Anspruchs 1. Alternativlösungen sind in den Ansprüchen 2 und 3 angegeben.

Alle drei vorgeschlagenen Lösungen sehen eine Gelenkendoprothese vor, welche ein Gelenkteil mit metallischem Kern aufweist, wobei der metallische Kern in Bereichen, die an einem anderen Gelenkteil bei Ausübung der Gelenkfunktion abrollen und/oder abgleiten, beschichtet ist mit einer Glaskeramik-Verbundmischung unterschiedlicher Zusammensetzungen.

Gemäß der ersten Lösung wird eine nachstehend wiedergegebene Zusammensetzung vorgeschlagen:

| | |
|---|---|
| SiO₂ | 45,0 - 50% |
| Al₂O₃ | 15,0 - 20,0% |
| ZrO₂ | 8,0 - 12,0% |
| K₂O | 8,0% |
| Na₂O | 7,5% |
| CaO | 2,5% |
| TiO₂ | 1,5% |

und darüber hinaus eine Restmenge von bis zu 12,5% aus MnO₂ und/oder Fe₂O₃ und/oder Co₂O₃.

Größter Anteil bei dieser Zusammensetzung ist der Glasanteil SiO₂, bei dessen Verwendung das Anhaften am metallischen Untergrund im Vordergrund steht. Das Abriebsverhalten wird maßgeblich beeinflusst durch die Komponenten Al₂O₃ und ZrO₂.

Diese Zusammensetzung der Glaskeramik ist die härteste der drei Lösungsvorschläge. Es wird als so genanntes bionisches System bezeichnet, da es ermöglicht, dass das andere Gelenkteil mit ebenfalls einem metallischen Kern mit einer Glaskeramik-Verbundmischung derselben Zusammensetzung beschichtet ist. Es werden Laufzeiten der Gelenkendoprothese, welche die beschriebene Glaskeramik-Zusammensetzung trägt, von 15 bis 20 Jahren erwartet.

Gemäß einem zweiten Lösungsvorschlag ist die folgende Zusammensetzung der Glaskeramik vorgesehen:

| | |
|---|---|
| SiO₂ | 35,0 - 55,0% |
| Al₂O₃ | 15,0 - 25,0% |
| TiO₂ | 10,0 - 14,0% |
| Na₂O | 7,5% |
| K₂O | 7,0% |
| ZrO₂ | 1,0 - 5,0% |
| CaO | 0,5% |

und darüber hinaus eine Restmenge von bis zu 24% aus MnO₂ und/oder Fe₂O₃ und/oder Co₂O₃ und /oder CuO.

Dieses System unterscheidet sich vom ersten Lösungsvorschlag zunächst einmal darin, dass die Beschichtung etwas weicher ist als die erstgenannte. Entscheidender Unterschied ist aber die wesentlich höhere Beimengung von TiO₂. Damit ist ein zwar geringer, aber nie zu vermeidender Abrieb körperverträglicher. Daher wird dieses System auch als antiallergen bezeichnet. Die Laufzeiten einer damit ausgestatteten Gelenkendoprothese dürften etwas niedriger ausfallen als bei der ersten Gelenkendoprothese. Auch dieses System dieser Glaskeramik-Verbundmischung ist so hart, dass auch das andere Gelenkteil mit ebenfalls einem metallischen Kern mit der Glaskeramik-Verbundmischung derselben Zusammensetzung wie das erste Gelenkteil beschichtet sein kann.

Gemäß dem dritten Vorschlag ist das erste Gelenkteil mit einer Glaskeramik der folgenden Zusammensetzung beschichtet:

| | |
|---|---|
| SiO₂ | 50,0 - 60,0% |
| Al₂O₃ | 5,0 - 10,0% |
| ZrO₂ | 5,0 - 10,0% |
| Na₂O | 8,0% |
| CaO | 6,0% |
| TiO₂ | 3,0 - 5,0% |
| K₂O | 2,0% |
| Sb₂O₅ | 0,5% |

und darüber hinaus eine Restmenge von bis zu 20,5% aus MnO₂ und/oder Fe₂O₃ und/oder Co₂O₃ und /oder CuO und/oder CeO₂.

Diese Variante stellt gemessen an den beiden anderen Varianten das weichste Material zur Verfügung. Gleichwohl werden Laufzeiten von bis zu 15 Jahren erwartet. Aufgrund der relativen Weichheit des Materials wird bevorzugt vorgesehen, dass das andere Gelenkteil, an welchem das erste Gelenkteil abrollt und/oder daran abgleitet, einen Gleitpartner aus einer Keramik aufweist, die härter ist als die Glaskeramik-Verbundmischung vorerwähnter Zusammensetzung. Besonders bevorzugt wird in diesem Falle als Gleitpartner eine Komposit-Keramik, wie sie beispielsweise bekannt ist aus der EP 0 502 082 C. Alternativ kann hierzu bei dem zweiten Gelenkteil ein Gleitpartner aus Polyäthylen zur Anwendung kommen. Die so aufgebaute Gelenkendoprothese kann als Standard-Endoprothese bezeichnet werden, die dem Durchschnittspatienten implantiert wird. Gleichwohl hat sie gegenüber den bekannten Gelenkendoprothesen mit den Paarungen Metall gegen Metall, Metall gegen Polyäthylen, Keramik gegen Polyäthylen etc. eine längere Laufzeit bei einer Festigkeit der Komponenten, die deutlich höher als bei insbesondere keramischen Bauteilen ist.

Besonders bevorzugt wird, wenn die metallischen Kerne der Gelenkteile aus einer Kobalt-Chrom-Molybdän-Legierung bestehen. Es hat sich nämlich herausgestellt, dass die Haftkräfte der Beschichtungen auf diesem Metall extrem hoch sind, so dass eine Beschädigung der Beschichtung, beispielsweise ein Abplatzen höchst unwahrscheinlich ist.

Bei allen Varianten wird bevorzugt, wenn die Dicke der Beschichtung 200µm bis 600µm beträgt. Bei einem durchschnittlichen Abrieb von 5µm bis 8µm pro Jahr ergeben sich hieraus extrem lange Laufzeiten, die mit Materialpaarungen gemäß dem Stand der Technik schwer zu erzielen sind.

## Patentansprüche

1. Gelenkendoprothese, aufweisend ein Gelenkteil mit metallischem Kern, wobei der metallische Kern in Bereichen, die an einem anderen Gelenkteil bei Ausübung der Gelenkfunktion abrollen und/oder abgleiten, beschichtet ist mit einer Glaskeramik der folgenden Zusammensetzung (in Gew.-%)
| | |
|---|---|
| SiO₂ | 45,0 - 50% |
| Al₂O₃ | 15,0 - 20,0% |
| ZrO₂ | 8,0 - 12,0% |
| K₂O | 8,0% |
| Na₂O | 7,5% |
| CaO | 2,5% |
| TiO₂ | 1,5% |
und darüber hinaus eine Restmenge von bis zu 12,5% aus MnO₂ und/oder Fe₂O₃ und/oder Co₂O₃.

2. Gelenkendoprothese, aufweisend ein Gelenkteil mit metallischem Kern, wobei der metallische Kern in Bereichen, die an einem anderen Gelenkteil bei Ausübung der Gelenkfunktion abrollen und/oder abgleiten, beschichtet ist mit einer Glaskeramik der folgenden Zusammensetzung (in Gew.-%)
| | |
|---|---|
| SiO₂ | 35,0 - 55,0% |
| Al₂O₃ | 15,0 - 25,0% |
| TiO₂ | 10,0 -14,0% |
| Na₂O | 7,5% |
| K₂O | 7,0% |
| ZrO₂ | 1,0 - 5,0% |
| CaO | 0,5% |
und darüber hinaus eine Restmenge von bis zu 24% aus MnO₂ und/oder Fe₂O₃ und/oder Co₂O₃ und /oder CuO.

3. Gelenkendoprothese, aufweisend ein Gelenkteil mit metallischem Kern, wobei der metallische Kern in Bereichen, die an einem anderen Gelenkteil bei Ausübung der Gelenkfunktion abrollen und/oder abgleiten, beschichtet ist mit einer Glaskeramik der folgenden Zusammensetzung (in Gew.-%)
| | |
|---|---|
| SiO₂ | 50,0 - 60,0% |
| Al₂O₃ | 5,0 - 10,0% |
| ZrO₂ | 5,0 -10,0% |
| Na₂O | 8,0% |
| CaO | 6,0% |
| TiO₂ | 3,0 - 5,0% |
| K₂O | 2,0% |
| Sb₂O₅ | 0,5% |
und darüber hinaus eine Restmenge von bis zu 20,5% aus MnO₂ und/oder Fe₂O₃ und/oder Co₂O₃ und /oder CuO und/oder CeO₂ .

4. Gelenkendoprothese nach einem der Ansprüche 1 bis 3, bei dem die Dicke der Beschichtung 200 bis 600 µm beträgt.

5. Gelenkendoprothese nach Anspruch 1 oder 2, bei der das andere Gelenkteil ebenfalls einen metallischen Kern aufweist, der mit einer Glaskeramik-Verbundmischung derselben Zusammensetzung wie das erste Gelenkteil beschichtet ist.

6. Gelenkendoprothese nach Anspruch 3, bei der das andere Gelenkteil einen Gleitpartner aus einer Keramik aufweist.

7. Gelenkendoprothese nach Anspruch 3, bei der das andere Gelenkteil einen Gleitpartner aus Polyäthylen aufweist.

8. Gelenkendoprothese nach Anspruch 1 bis 7, bei dem die metallischen Kerne der Gelenkteile aus einer Kobalt-Chrom-Molybdän-Legierung bestehen.

## Claims

1. Joint endoprosthesis having a joint part with metallic core, wherein the metallic core is coated, in regions which roll off and/or slide off on a different joint part when exerting the joint function, with a glass ceramic of the following composition (in wt.%)
| | |
|---|---|
| SiO₂ | 45.0 - 50% |
| Al₂O₃ | 15.0 - 20.0% |
| ZrO₂ | 8.0 - 12.0% |
| K₂O | 8.0% |
| Na₂O | 7.5% |
| CaO | 2.5% |
| TiO₂ | 1.5% |
and furthermore a remaining quantity of up to 12.5% of MnO₂ and/or Fe₂O₃ and/or Co₂O₃.

2. Joint endoprosthesis having a joint part with metallic core, wherein the metallic core is coated, in regions which roll off and/or slide off on a different joint part when exerting the joint function, with a glass ceramic of the following composition (in wt.%)
| | |
|---|---|
| SiO₂ | 35.0 - 55.0% |
| Al₂O₃ | 15.0-25.0% |
| TiO₂ | 10.0 - 14.0% |
| Na₂O | 7.5% |
| K₂O | 7.0% |
| ZrO₂ | 1.0 - 5.0% |
| CaO | 0.5% |
and furthermore a remaining quantity of up to 24% of MnO₂ and/or Fe₂O₃ and/or Co₂O₃ and/or CuO.

3. Joint endoprosthesis having a joint part with metallic core, wherein the metallic core is coated, in regions which roll off and/or slide off on a different joint part when exerting the joint function, with a glass ceramic of the following composition (in wt.%)
| | |
|---|---|
| SiO₂ | 50.0 - 60.0% |
| Al₂O₃ | 5.0 - 10.0% |
| ZrO₂ | 5.0 - 10.0% |
| Na₂O | 8.0% |
| CaO | 6.0% |
| TiO₂ | 3.0 - 5.0% |
| K₂O | 2.0% |
| Sb₂O₅ | 0.5% |
and furthermore a remaining quantity of up to 20.5% of MnO₂ and/or Fe₂O₃ and/or Co₂O₃ and/or CuO and/or CeO₂.

4. Joint endoprosthesis according to one of claims 1 to 3, in which the thickness of the coating is 200 to 600 µm.

5. Joint endoprosthesis according to claim 1 or 2, in which the other joint part likewise has a metallic core which is coated with a glass-ceramic compound mixture of the same composition as the first joint part.

6. Joint endoprosthesis according to claim 3, in which the other joint part has a sliding partner made from a ceramic.

7. Joint endoprosthesis according to claim 3, in which the other joint part has a sliding partner made from polyethylene.

8. Joint endoprosthesis according to claim 1 to 7, in which the metallic cores of the joint parts consist of a cobalt-chromium-molybdenum alloy.

## Revendications

1. Endoprothèse articulaire, comportant une partie d'articulation avec noyau métallique, le noyau métallique étant revêtu, dans des zones qui roulent et/ou glissent sur une autre partie d'articulation lors de l'exercice de la fonction de l'articulation, d'une vitrocéramique de la composition suivante (en % de poids)
| | |
|---|---|
| SiO₂ | 45,0 - 50 % |
| Al₂O₃ | 15,0 - 20,0 % |
| ZrO₂ | 8,0 - 12,0 % |
| K₂O | 8,0 % |
| Na₂O | 7,5 % |
| CaO | 2,5 % |
| TiO₂ | 1,5 % |
et, en outre, d'une quantité résiduelle de jusqu'à 12,5 % de MnO₂ et/ou de Fe₂O₃ et/ou de CO₂O₃.

2. Endoprothèse articulaire, comportant une partie d'articulation avec noyau métallique, le noyau métallique étant revêtu dans des zones qui roulent et/ou glissent sur une autre partie d'articulation lors de l'exercice de la fonction de l'articulation, d'une vitrocéramique de la composition suivante (en % de poids)
| | |
|---|---|
| SiO₂ | 35,0 - 55,0 % |
| Al₂O₃ | 15,0 - 25,0 % |
| TiO₂ | 10,0 - 14,0 % |
| Na₂O | 7,5 % |
| K₂O | 7,0 % |
| ZrO₂ | 1,0 - 5,0 % |
| CaO | 0,5 % |
et, en outre, d'une quantité résiduelle de jusqu'à 24 % de MnO₂ et/ou de Fe₂O₃ et/ou de CO₂O₃ et/ou de CuO.

3. Endoprothèse articulaire, comportant une partie d'articulation avec noyau métallique, le noyau métallique étant revêtu dans des zones qui roulent et/ou glissent sur une autre partie d'articulation lors de l'exercice de la fonction de l'articulation, d'une vitrocéramique de la composition suivante (en % de poids)
| | |
|---|---|
| SiO₂ | 50,0 - 60, % |
| Al₂O₃ | 5,0 - 10,0 % |
| ZrO₂ | 5,0 - 10,0 % |
| Na₂O | 8,0 % |
| CaO | 6,0 % |
| TiO₂ | 3,0 - 5,0 % |
| K₂O | 2,0 % |
| Sb₂O₅ | 0,5 % |
et, en outre, d'une quantité résiduelle de jusqu'à 20,5 % de MnO₂ et/ou de Fe₂O₃ et/ou de CO₂O₃ et/ou de CeO₂.

4. Endoprothèse articulaire selon une des revendications 1 à 3, dont l'épaisseur du revêtement est de 200 à 600 µm.

5. Endoprothèse articulaire selon la revendication 1 ou 2, où l'autre partie d'articulation comporte également un noyau métallique qui est revêtu d'un mélange composite en vitrocéramique de composition identique à celui utilisé pour la première partie de l'articulation.

6. Endoprothèse articulaire selon la revendication 3, où l'autre partie d'articulation comporte un partenaire de glissement en céramique.

7. Endoprothèse articulaire selon la revendication 3, où l'autre partie d'articulation comporte un partenaire de glissement en polyéthylène.

8. Endoprothèse articulaire selon l'une des revendications 1 à 7, où les noyaux métalliques des parties d'articulations sont composés d'un alliage cobalt-chrome-molybdène.
